# EUROPÄISCHE PATENTSCHRIFT

(11) **EP 0 827 537 B1**
(45) Veröffentlichungstag und Bekanntmachung des Hinweises auf die Patenterteilung: **13.08.2008**
(21) Anmeldenummer: 96914165.4
(22) Anmeldetag: 02.05.1996
(51) Int. Cl.: C12N 15/12, C07K 14/47, C07K 16/18, C12N 15/11, G01N 33/50, A01K 67/00, C12N 1/21, C12N 5/10

(54) **CHROMATIN-REGULATORGENE**
CHROMATIN-REGULATOR GENES
GENES DE REGULATION DE LA CHROMATINE

(30) Priorität: 10.05.1995 DE 19516776
(43) Veröffentlichungstag der Anmeldung: 11.03.1998
(73) Patentinhaber: Boehringer Ingelheim International GmbH, 55218 Ingelheim am Rhein (DE)
(72) Erfinder: JENUWEIN, Thomas, A-1030 Wien (AT); LAIBLE, Götz, A-1150 Wien (AT)
(86) Internationale Anmeldenummer: PCT/EP1996/001818
(87) Internationale Veröffentlichungsnummer: WO 1996/035784

(56) Entgegenhaltungen:
- DATABASE EMBL entry HS529651, accession number U52965, 26.April 1996 HOBERT O ET AL: "Interaction of Vav with ENX-q, a putative transcriptional regulator of homeobox gene expression" XP002017786 & MOLECULAR AND CELLULAR BIOLOGY, Bd. 16, Nr. 6, Juni 1996, WASHINGTON US, Seiten 3066-3073, XP000609032 HOBERT O ET AL: "Interaction of Vav with ENX-1, a putative transcriptional regulator of homeobox gene expression"
- DATABASE EMBL entry HSMG44A, accession number L08238, Januar 1993 GERAGHTY MT: "Human MG44 mRNA, 5' end" XP002017787 in der Anmeldung erwähnt & GENOMICS, Bd. 16, 1993, Seiten 440-446, XP000609031 GERAGHTY ET AL: "The isolation of cDNAs from OATL1 at Xp11.2 using a 480-kb YAC" in der Anmeldung erwähnt
- DATABASE EMBL entry HS18003, accession number U18003, Dezember 1994 OSTERMEYER EA: "Human chromosome 17q21 mRNA clone B117" XP002017911 & GENOMICS, Bd. 25, Januar 1995, Seiten 256-263, XP000609030 FRIEDMAN LS: "22 genes from chromosome 17q21: cloning, sequencing and characterization of mutations in breast cancer families and tumors"
- MOLECULAR AND CELLULAR BIOLOGY, Bd. 13, 1993, WASHINGTON US, Seiten 6357-6366, XP000608922 JONES RS ET AL: "The Drosophila polycomb-group gene enhancer of zeste contains a region with sequence similarity to trithorax" in der Anmeldung erwähnt
- EMBO JOURNAL, Bd. 13, 1994, EYNSHAM, OXFORD GB, Seiten 3822-3831, XP002017785 TSCHIERSCH B ET AL: "The protein encoded by the Drosophila position-effect variegation suppressor gene Su(var)3-9 combines domains of antagonistic regulators of homeotic gene complexes" in der Anmeldung erwähnt

## Beschreibung

Die vorliegende Erfindung bezieht sich auf Gene, die eine Rolle bei der strukturellen und funktionellen Regulation von Chromatin spielen, und ihre Verwendung für die Therapie und Diagnostik.

Die funktionelle Organisation von eukaryotischen Chromosomen in Centromere, Telomere sowie in eu- und heterochromatische Regionen stellt einen entscheidenden Mechanismus zur Gewährleistung der genauen Replikation und Verteilung der genetischen Information bei jeder Zellteilung dar. Im Gegensatz dazu sind Tumorzellen häufig durch chromosomale Rearrangements, Translokationen und Aneuploidie charakterisiert (Solomon et al., 1991; Pardue, 1991). Obwohl die Mechanismen, die zu einer erhöhten Chromosomeninstabilität in Tumorzellen führen, noch nicht geklärt sind, haben es in jüngster Zeit eine Reihe von experimentellen Systemen, beginnend mit telomerischen Positionseffekten in Hefe (Renauld et al., 1993; Buck und Shore, 1995; Allshire et al., 1994), über Positionseffekt-Variegation (PEV) in *Drosophila* (Reuter und Spierer, 1992), bis zur Analyse von Translokationsbruchpunkten in humanen Leukämien (Solomon et al., 1991; Cleary, 1991), ermöglicht, einige chromosomale Proteine zu identifizieren, die an der deregulierten Proliferation ursächlich beteiligt sind.

Erstens wurde festgestellt, daß die Überexpression einer verkürzten Version des SIR4-Proteins zu einer verlängerten Lebensdauer in Hefe führt (Kennedy et al., 1995). Da SIR-Proteine zum Entstehen multimerer Komplexe an den stillen "Mating Type Loci" und am Telomer beitragen, könnte es sein, daß überexprimiertes SIR4 mit diesen heterochromatinartigen Komplexen interferiert, was schließlich zu einer unkontrollierten Proliferation führt. Diese Annahme steht im Einklang mit der Häufigkeit des Auftretens einer deregulierten Telomerenlänge in den meisten humanen Krebsarten (Counter et al., 1992).

Zweitens wurden mittels genetischer Analysen von PEV in *Drosophila* eine Reihe von Genprodukten identifiziert, die die Chromatinstruktur an heterochromatischen Positionen und innerhalb des homeotischen Genclusters verändern (Reuter und Spierer, 1992). Mutationen einiger dieser Gene, wie *modulo* (Garzino et al., 1992) und *polyhomeotic* (Smouse und Perrimon, 1990), können in *Drosophila* die deregulierte Zellproliferation oder den Zelltod verursachen. Drittens wurden Säugetierhomologe von sowohl Aktivatoren (*trithorax* oder trx-Gruppe) als auch Repressoren (z.B. *polycomb* oder Pc-Gruppe) der Chromatinstruktur von homeotischen *Drosophila-*Selektorgenen beschrieben. Unter diesen hat sich vom humanen *HRX*/*ALL-1* (*trx*-Gruppe) gezeigt, daß es an der durch Translokation induzierten Leukämogenese beteiligt ist (Tkachuk et al., 1992; Gu et al., 1992), und daß die Überexpression des Maus-*bmi* (*Pc*-Gruppe) zum Entstehen von Lymphomen führt (Haupt et al., 1991; Brunk et al., 1991; Alkema et al., 1995). Ein Modell für die Funktion chromosomaler Proteine läßt darauf schließen, daß diese multimere Komplexe bilden, welche in Abhängigkeit von der Ausgewogenheit zwischen Aktivatoren und Repressoren im Komplex den Kondensationsgrad der umliegenden Chromatinregion bestimmen (Locke et al., 1988). Eine Verschiebung dieses Gleichgewichts durch Überexpression einer der Komponenten des Komplexes zeigte eine Neuverteilung von eu- und heterochromatischen Regionen (Buck und Shore, 1995; Reuter und Spierer, 1992; Eissenberg et al., 1992). Dieser Dosiseffekt kann die Chromatinstruktur an vorbestimmten Loci destabilisieren, was letztlich zu einem Übergang vom normalen zum transformierten Zustand führt.

Trotz der Charakterisierung von *HRX*/*ALL-1* und *bmi* als Protoonkogene, die die Chromatinstruktur verändern können, ist das Wissen über Säugetiergenprodukte, welche mit Chromatin wechselwirken, noch sehr beschränkt. Im Gegensatz dazu wurden durch genetische Analysen von PEV in *Drosophila* ca. 120 Allele für Chromatinregulatoren beschrieben (Reuter und Spierer, 1992). Kürzlich wurde eine carboxyterminale Region mit Ähnlichkeit in der Sequenz identifiziert, die einem positiven (*trx*, *trx*-Gruppe) und einem negativen (*E*(*z*), *Pc*-Gruppe) *Drosophila*-Chromatinregulator (Jones und Gelbart, 1993) gemeinsam ist. Darüberhinaus ist dieser Carboxyterminus auch in *Su*(*var*)3-9, einem dominanten Supressor der Chromatinverteilung in *Drosophila*, konserviert (Tschiersch et al., 1994).

Bei der vorliegenden Erfindung wurde von der Überlegung ausgegangen, daß diese als "SET" bezeichnete Proteindomäne (Tschiersch et al., 1994) wegen ihrer evolutionären Konservierung und dem Vorhandensein in antagonistischen Genprodukten eine neue Genfamilie entwicklungsgeschichtlich wichtiger Säugetier-Chromatinregulatoren definiert. Darüberhinaus trägt die Charakterisierung weiterer Mitglieder der Gruppe von SET-Domäne-Genen, neben *HRX*/*ALL-1*, zur Aufklärung der Mechanismen bei, die dafür verantwortlich sind, daß strukturelle Veränderungen im Chromatin zur malignen Transformation führen können.

Der vorliegenden Erfindung lag somit die Aufgabe zugrunde, humane Chromatinregulatorgene zu identifizieren, ihre Funktion aufzuklären und sie für die Diagnostik und Therapie einzusetzen.

Zur Lösung dieser Aufgabe wurde zunächst die Sequenzinformation der SET-Domäne benutzt, um aus humanen cDNA-Banken die zu den SET-Domäne-Genen von Drosophila homologen humanen cDNAs zu erhalten. Es wurden zwei cDNAs erhalten, die Humanhomologe von E(z) bzw. Su(var)3-9 darstellen; die entsprechenden humanen Gene wurden als EZH2 und SUV39H bezeichnet (vgl. unten); außerdem wurde eine variante Form von EZH2 identifiziert, die als EZH1 bezeichnet wurde.

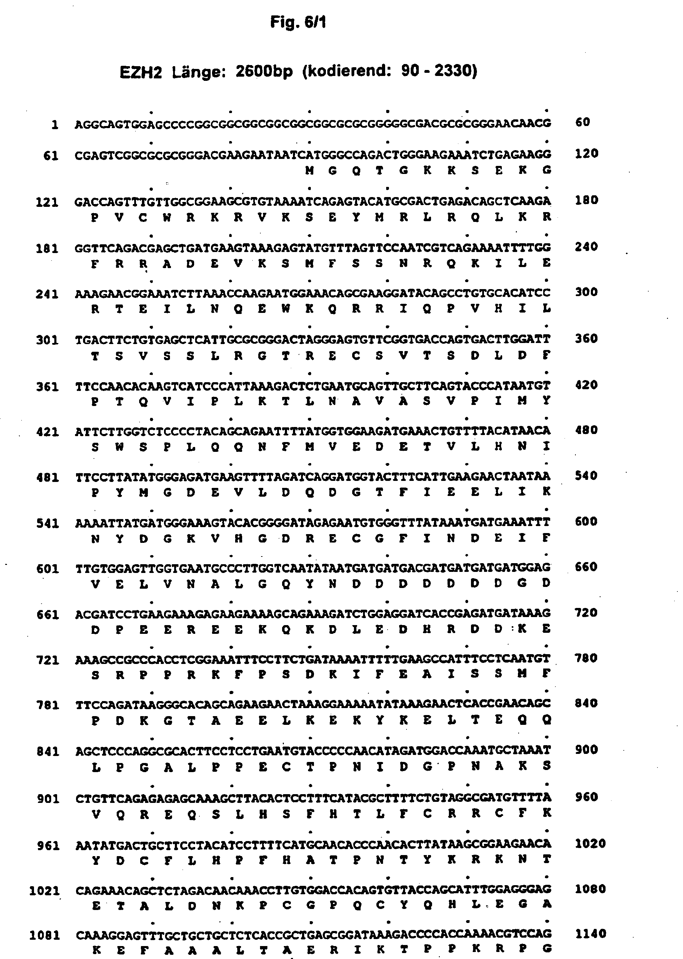

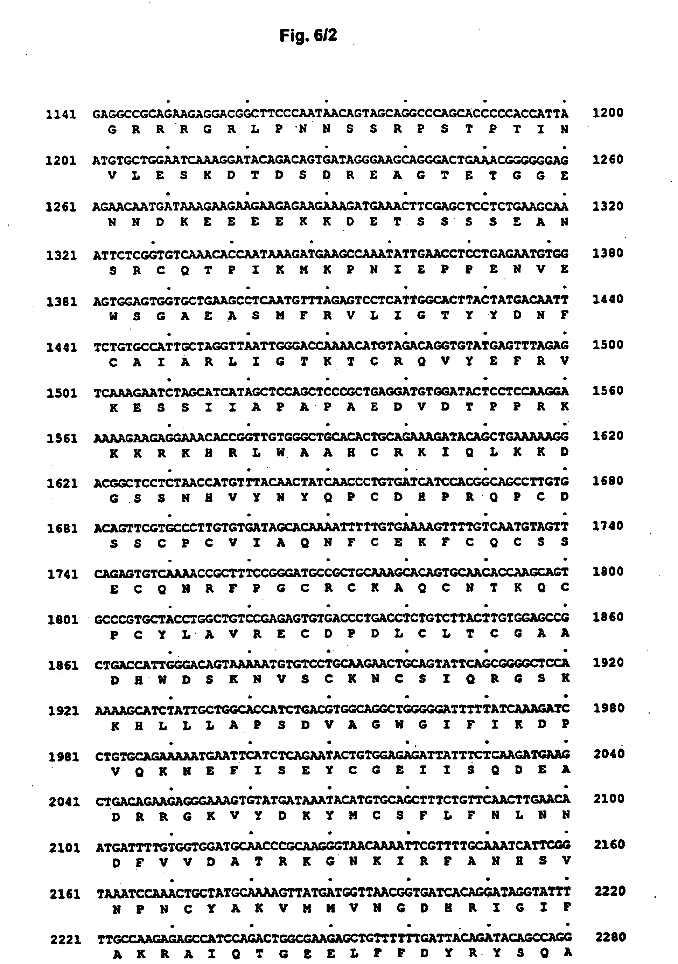

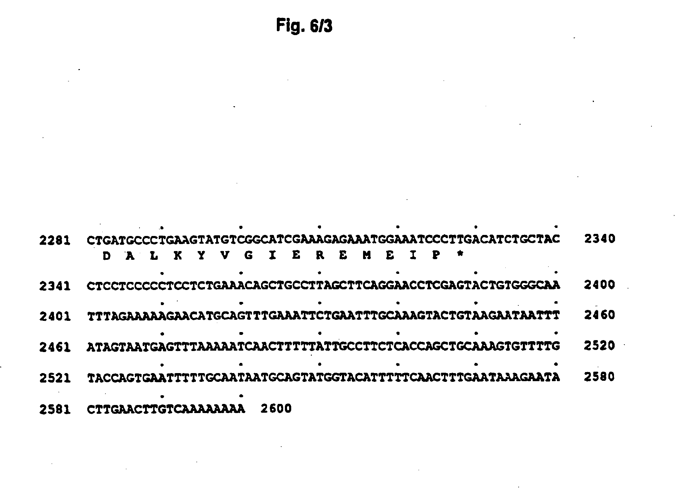

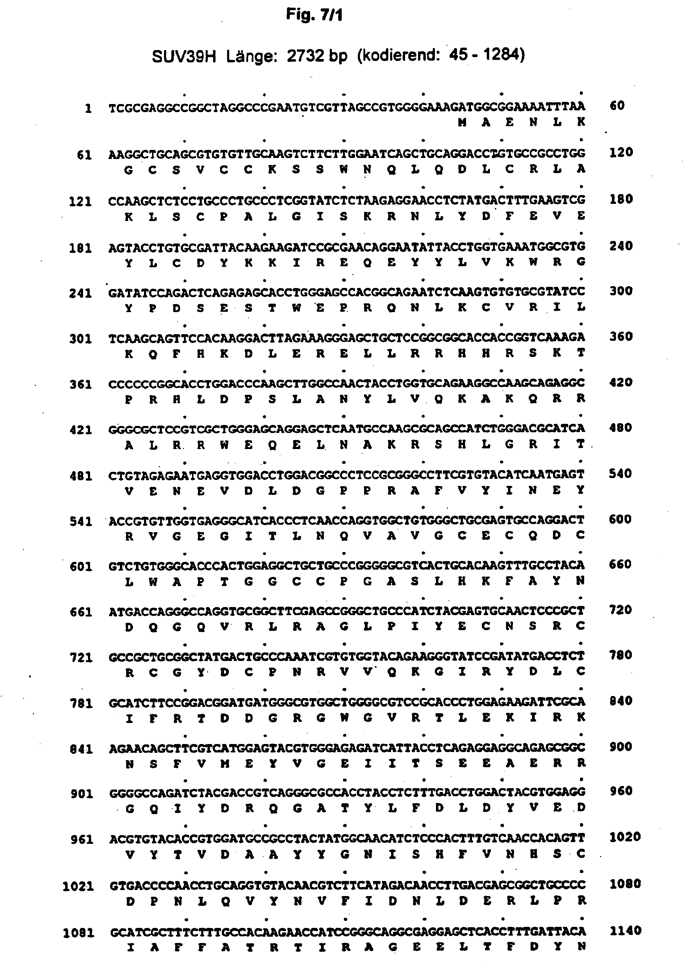

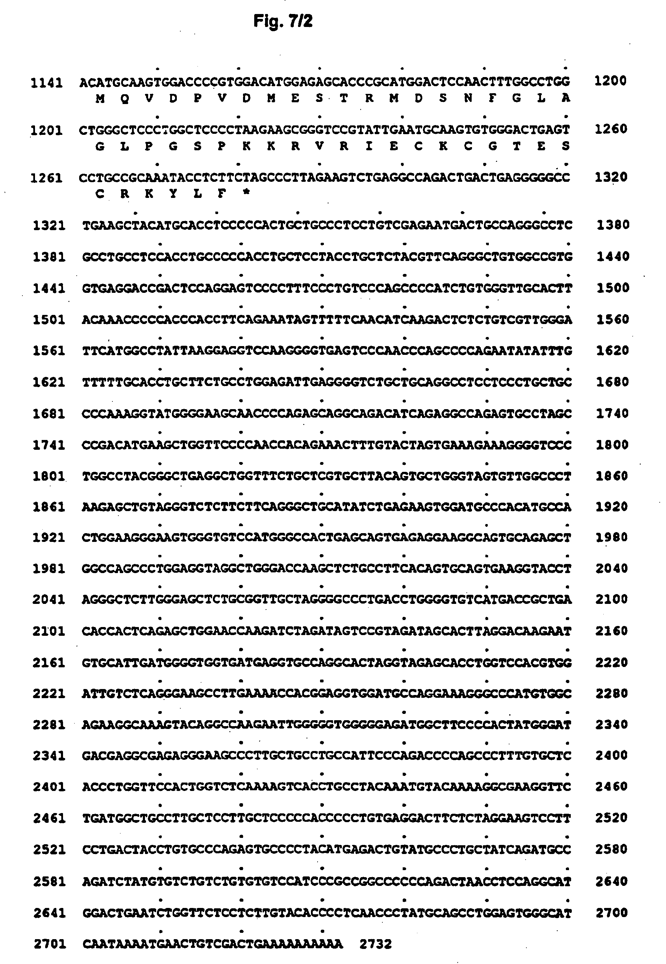

Die vorliegende Erfindung betrifft somit ein DNA-Molekül, kodierend für ein humanes Chromatinregulator-Protein oder für eine Teilsequenz davon, die der SET-Domäne entspricht, dadurch gekennzeichnet, dass es ausgewählt ist aus der Gruppe von DNA-Molekülen, welche die in Fig. 6 bzw. Fig. 7 dargestellte Nukleotidsequenz aufweisen, kodierend für Proteine der Bezeichnung EZH2 bzw. SUV39H, oder Abschnitte dieser Sequenz, die der SET-Domäne von EZH2 bzw. SUV39H entsprechen.

Die erfindungsgemäßen Gene tragen die Bezeichnung EZH2 und SUV39H, sie wurden ursprünglich als "HEZ-2" und "H3-9" bezeichnet.

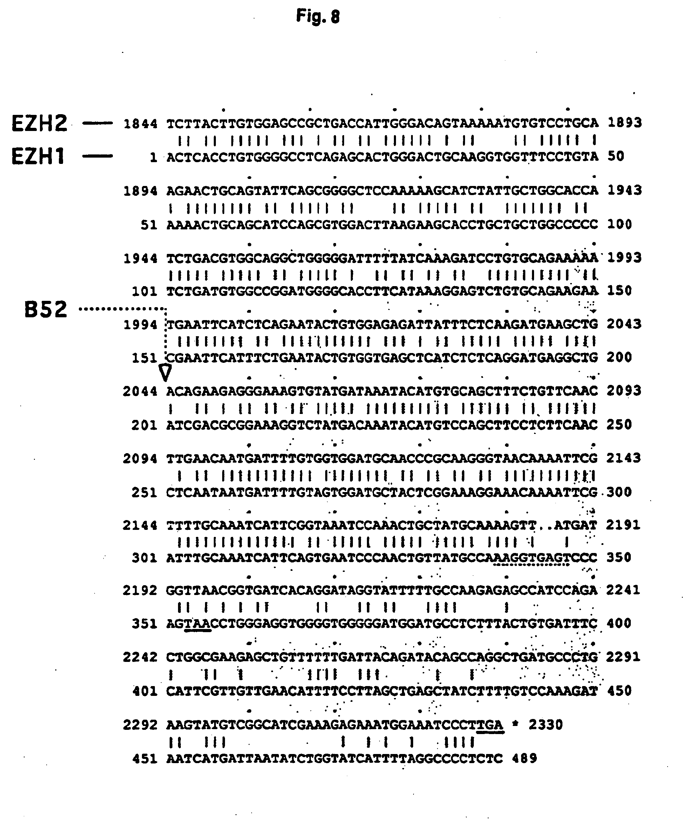

Die Erfindung betrifft in einem weiteren Aspekt die von diesen Genen abgeleiteten cDNAs, einschließlich ihrer degenerierten Varianten; Mutanten, die für funktionelle Chromatinregulatoren kodieren sowie eine Variante, die auf eine Genduplikation zurückzuführen sind (EZH1, dessen Teilsequenz im Vergleich mit EZH2 in Fig. 8 dargestellt ist).

Zur Lösung der im Rahmen der vorliegenden Erfindung gestellten Aufgabe wurde im einzelnen wie folgt vorgegangen: Ausgehend von der Sequenzinformation von der konservierten SET-Domäne, wurde unter reduzierter Stringenz eine humane B-Zell-spezifische cDNA-Bibliothek mit einer gemischten *Drosophila*-DNA-Sonde, die für die SET-Domänen von *E*(*z*) und *Su*(*var*)3-9 kodiert, gescreent. Aus 500.000 Plaques wurden 40 primäre Phagen ausgewählt. Nach zwei weiteren Runden Screening zeigte sich, daß 31 Phagen für authentische *E*(*z*)-Sequenzen kodieren und daß fünf Phagen *E*(*z*)-Varianten darstellen. Im Gegensatz dazu hybridisierten nur zwei Phagen mit der Sonde, die die SET-Domäne von *Su*(*var*)3-9 allein enthielt. Die Phageninserts wurden mittels Polymerasekettenreaktion (PCR) amplifiziert und mittels Restriktionskartierung und Teilsequenzierung analysiert. Repräsentative cDNA-Inserts wurden subkloniert und über ihre ganze Länge sequenziert. Die 5'-Enden wurden isoliert, indem positive Phagen noch einmal mit 5'-DNA-Sonden gescreent wurden, worauf nach Subklonierung komplette cDNAs erhalten wurden.

Die komplette, für das humane Homologe von *E*(*z*) kodierende cDNA wurde als *EZH2* und die DNA, die für das humane Homologe von *Su*(*var*)3-9 kodiert, wurde als *SUV39H* bezeichnet. Insgesamt beträgt die Identität der Aminosäuren zwischen *Drosophila* und den humanen Proteinen 61 % für *EZH2* und 43 % für *SUV39H*, wobei die C-terminale SET-Domäne sehr hoch konserviert ist (88 % für *EZH2* und 53 % für *SUV39H*). Der Sequenzvergleich zeigte weitere deutliche Homologieregionen, z.B. eine Cystein-reiche Domäne in *EZH2* und eine Chromo-Box in *SUV39H*. (In polycomb wurde gezeigt, daß die Chromo-Box die für die Wechselwirkung zwischen DNA und Chromatin wesentliche Domäne ist; Messmer et al., 1992). Im Gegensatz dazu fehlen die 207 Aminosäuren, die das aminoterminale GTP-Bindungsmotiv des Drosophila-Proteins enthalten, im humanen Homologen *SUV39H*.

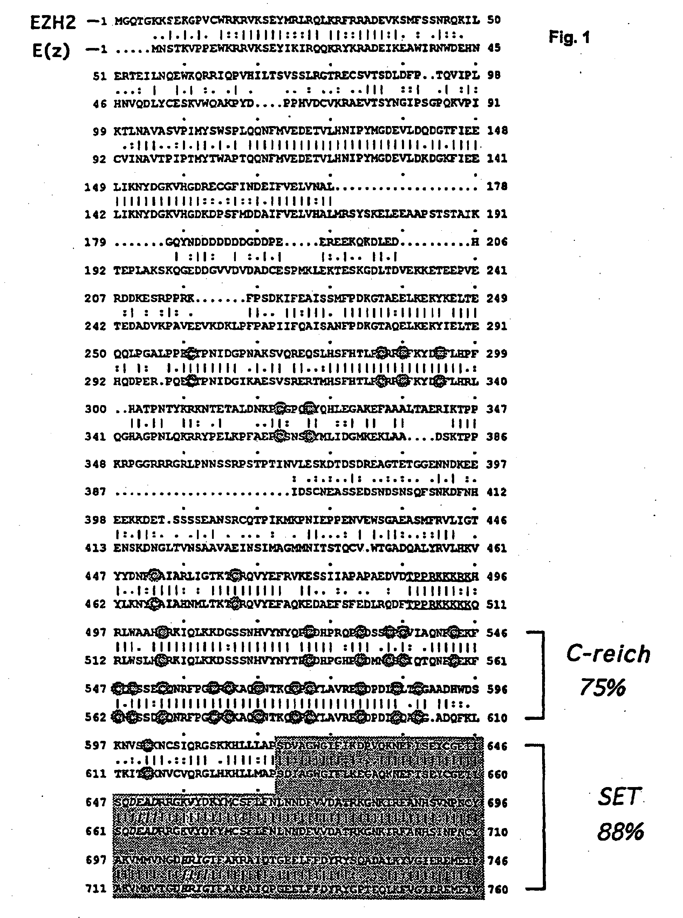

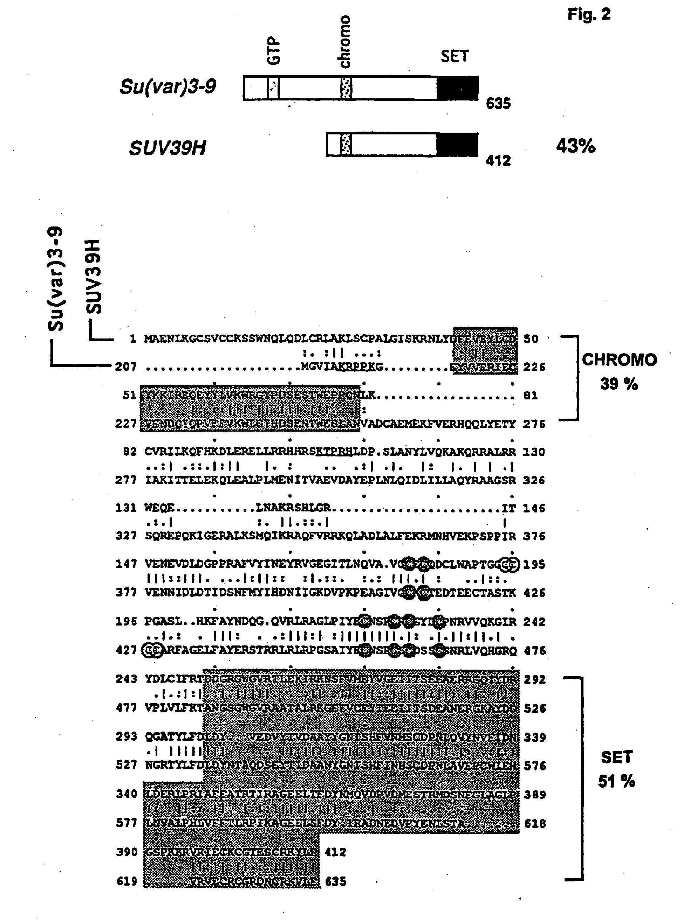

Der Vergleich der Aminosäure-Sequenzen zwischen den *Drosophila*- und den humanen Genen ist in den Fig. 1 und 2 dargestellt:
Fig. 1 zeigt den Vergleich der Aminosäure-Sequenz zwischen EZH2 und von *Drosophila Enhancer* von *zeste* (*E(z)*). Identische Aminosäuren der konservierten carboxyterminalen SET-Domäne (schattierte Box) und der Cys-reichen Region (Cys-Reste sind hervorgehoben) sind gezeigt. Die mutmaßlichen Kernlokalisierungssignale sind unterstrichen.
Fig. 2 zeigt den Vergleich der Aminosäure-Sequenz zwischen dem humanen Homologen *SUV39H* und *Drosophila Su*(*var*)3-9. Identische Aminosäuren der konservierten carboxyterminalen SET-Domäne (schattierte Box) und der Chromo-Domäne (dunkler schattierte Box) sind dargestellt. Die mutmaßlichen Kernlokalisierungssignale sind unterstrichen. Oben in der Figur ist eine schematische Übersicht der beiden Proteinstrukturen dargestellt, die zeigt, daß im humanen Homologen 207 Aminosäuren am N-Terminus fehlen.

Da translationale Consensussequenzen in Umgebung des Start-ATG der humanen *SUV39H*-cDNA auch an der entsprechenden internen Position in *Su*(*var*)3-9 vorhanden sind, dürfte das *Drosophila*-Protein zusätzliche Exons enthalten, die in einem späteren Stadium der Evolution für die Funktion entbehrlich wurden. (Die Richtigkeit dieser Hypothese kann bestätigt werden, indem die humane *SUV39H*-cDNA und komplette oder am 5'-Ende verkürzte cDNAs von *Su*(*var*)3-9 in *Drosophila* exprimiert werden. Darüberhinaus wurde eine weitere cDNA der Bezeichnung *MG-44* beschrieben (s. unten), der ebenfalls das 5'-Ende des *Drosophila-*Gens fehlt.) Zusätzlich zur humanen cDNA von *SUV39H* wurde auch der homologe Locus in der Maus (Suv39h; siehe unten) isoliert, dessen Sequenzanalyse und Promotorstruktur eindeutig die aminoterminale Verkürzung säugerhomologer Gene im Vergleich zu *Drosophila Su(var)3-9* bestätigt.

Im Rahmen der vorliegenden Erfindung durchgeführte DNA-Blot-Analysen deuten darauf hin, daß säugerhomologe Gene von *Su(var)3-9* in der Maus und im Menschen von einzelnen Loci repräsentiert werden, während säugerhomologe Gene von *E*(*z*) in der Maus und im Menschen durch zwei getrennte Loci kodiert sind. Der zweite humane Locus (als *EZH1* bezeichnet) wurde auch durch Charakterisierung einer kleinen Anzahl von cDNA-Varianten, die sich in ihren 3'-flankierenden Sequenzen von der Mehrzahl der aus der humanen cDNA-Bibliothek isolierten Klone unterscheiden, bestätigt. Die Unterschiede zwischen *EZH2* und *EZH1* im sequenzierten Bereich sind in Fig. 8 dargestellt: Die SET-Domäne von *EZH1* zeigt gegenüber *EZH2* Mutationen; außerdem trägt die von uns isolierte *EZH1*-Variante (sehr wahrscheinlich eine aberrant gespleißte cDNA) ein sich im Leserahmen befindliches Stopcodon, welches das Protein um die 47 C-terminalen Aminosäuren verkürzt. In Fig. 8 ist die Nukleotidsequenz der *EZH2*-cDNA von Position 1844 bis 2330 in der jeweils oberen Zeile dargestellt, wobei die 5' Spleißstelle und das potentielle Stopcodon unterstrichen sind. Um eine Teilsequenz der cDNA der *EZH1*-Variante der *EZH2*-Sequenz zuzuordnen, wurde das gap-Programm des Wisconsin GCG Netzwerkservice verwendet. Das vorzeitige Stopcodon in *EZH1* (Position 353) ist unterstrichen. Sequenzen, die für die konservierte SET-Domäne kodieren, sind hervorgehoben. Außerdem ist das 3'-Ende (Position 151 in *EZH1*) des aberranten Transkripts *B52* (s. unten) dargestellt. Über die verfügbare Sequenz zeigte sich *B52* zu 97 % identisch mit *EZH1* und zu 72 % identisch mit *EZH2*. Der Sequenzvergleich von *EZH1* mit *EZH2* sowie die Feststellung, daß beim Menschen und der Maus zwei getrennte *E*(*z*)-homologe Loci auftreten, lassen darauf schließen, daß bei Säugern eine Genduplikation aufgetreten ist.

In einem Vergleich mit cDNA-Sequenzen in der Datenbank GeneBank wurde überraschend festgestellt, daß bestimmte in der Datenbank eingetragene cDNA-Teilsequenzen, die von aberranten Transkripten in Tumorgeweben abgeleitet sind, mutierte Versionen der erfindungsgemäßen cDNAs darstellen:

Einerseits war auf der Suche nach BRCA1, einem Gen, das für Brust- und Eileiterkrebs prädisponiert, eine partielle cDNA-Sequenz mit 271 Nukleotiden der Bezeichnung *B52*, die für eine mutierte Variante der SET-Domäne kodiert, isoliert und auf dem humanen Chromosom 17q21 kartiert worden (Friedman et al., 1994). Es wurde im Rahmen der vorliegenden Erfindung überraschend festgestellt, daß B52 97 % Identität mit der erfindungsgemäßen *EZH1*-cDNA-Variante aufweist (vgl. oben); möglicherweise stellt *EZH1* ein Gen dar, dessen Reaktivierung bei der deregulierten Proliferation eine Rolle spielt.

Andererseits war eine cDNA (2.800 Nukleotide; *MG-44*) vom humanen Chromosom Xp11 isoliert worden (Geraghty et al., 1993), einer Region, die für degenerative Störungen der Netzhaut und Synovialsarkome prädisponiert. Es wurde überraschend festgestellt, daß diese cDNA 98 % Identität mit der erfindungsgemäßen *SUV39H*-cDNA aufweist.

Die im Rahmen der vorliegenden Erfindung bereitgestellten neuen Gene ermöglichen es somit, einen Zusammenhang zwischen bestimmten Krebserkrankungen und Mutationen in Chromatinregulatoren herzuleiten; im Falle der *MG-44*-cDNA konnte, da diese mehrere Punkt- und Frameshift-Mutationen aufweist, welche die Chromo- und SET-Domänen unterbrechen, erst anhand der erfindungsgemäßen *SUV39H*-cDNA ein Zusammenhang zwischen *Su*(*var*)3-9 und *MG-44* aufgeklärt werden.

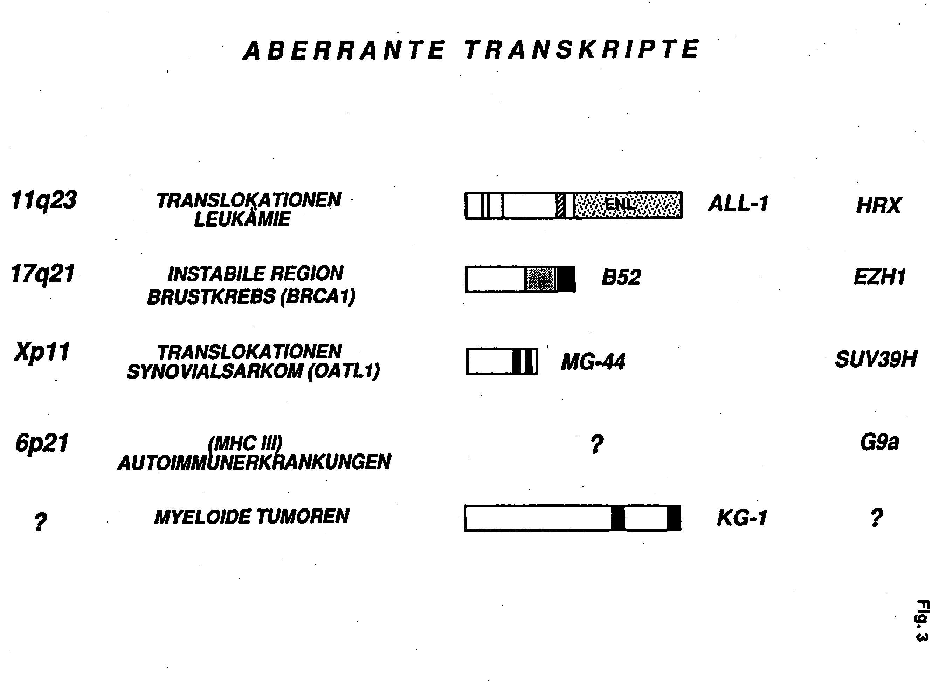

Neben den bereits genannten Sequenzen sind in der Sequenzdatenbank GeneBank als weitere humane Mitglieder der SET-Proteinfamilie das gut dokumentierte humane Homologe von *Drosophila trx, HRX*/*ALL-1* (Tkachuk et al., 1992; Gu et al., 1992) eingetragen, ferner ein Gen unbekannter Funktion der Bezeichnung G9a, das im humanen Major Histocompatibility Complex (Milner und Campbell, 1993) vorhanden ist, drittens eine nicht publizierte cDNA (*KG-1*), die aus unreifen myeloiden Tumorzellen isoliert wurde (Nomura et al., 1994). Während *G9a* derzeit das einzige humane Gen mit einer SET-Domäne ist, für das bisher keine mutierte Version bekannt ist, trägt *KG-1* eine Insertion von 342 Aminosäuren, welche die SET-Domäne in eine Amino- und eine Carboxyterminale Hälfte spaltet. Wahrscheinlich stellt diese *KG-1*-cDNA eine aberrant gespleißte Variante dar, da sich 5' und 3' Konsensus-Spleißstellen and beiden Enden der Insertion finden. Insgesamt sind vier der fünf derzeit bekannten humanen Mitglieder der SET-Proteinfamilie Änderungen unterworfen, die alle die SET-Domäne mutieren (*HRX*/*ALL-1, EZH1*/*B52, SUV39H*/*MG-44* und *KG-1*). Darüberhinaus wurden in drei Fällen die entsprechenden humanen Genloci in der Nähe von Translokationsbruchpunkten oder instabilen chromosomalen Regionen kartiert (*HRX*/*ALL-1, EZH1*/*B52* und *SUV39H*/*MG-44*). In Fig. 3 sind die aberranten Transkripte von humanen SET-Domäne-Genen dargestellt. Links in der Figur ist die Lage der fünf derzeit bekannten SET-Domäne-Gene auf dem jeweiligen Chromosom angegeben. U.a. sind die drei Gene (*HRX*/*ALL-1, ZZK1*/*B52* und *SUV39H*/*MG-44*), für die aberrante cDNAs auf Translokationsbruchpunkten oder instabilen Chromatinregionen kartiert wurden, dargestellt. Vier der fünf dargestellten SET-Domänen Gene weisen Mutationen auf, die alle die Carboxyterminale SET-Domäne unterbrechen, welche in der Figur durch die dunkle Box dargestellt ist. Eine Translokation verbindet die aminoterminale Hälfte von HRX mit einer nicht korrelierten Gensequenz, die als gepunktete Box mit der Bezeichnung ENL dargestellt ist. Mutationen und ein vorzeitiges Stopcodon verändern die SET-Domäne von *EZH1*/*B52*. Punkt- und Frameshift-Mutationen unterbrechen die Chromo- und SET-Domäne in *MG-44*. Eine große Insertion spaltet die SET-Domäne von *KG-1* in zwei Hälften. Aberrante Transkripte sind derzeit für G9a nicht bekannt. Das Cystein-reiche Cluster in *B52* ist als gepunktete Box dargestellt; in *HRX*/*ALL-1* sind die Homologieregion zu Methyltransferasen als schraffierte Box und die A/T-Haken als vertikale Linien dargestellt. Die Namen der jeweiligen authentischen Gene sind rechts in der Figur angegeben.

Die Tatsache, daß ein Säugetiergen der SET-Proteinfamilie, *HRX*/*ALL-1*, mit translokationsinduzierter Leukämogenese im Zusammenhang gebracht wurde (Tkachuk et al., 1992; Gu et al., 1992), ist ein starker Hinweis dafür, daß Proteine mit der SET-Domäne nicht nur wichtige Regulatoren der Entwicklung sind, die chromatinabhängige Veränderungen der Genexpression mitbestimmen, sondern daß sie, nach Mutation, auch die normale Zellproliferation stören.

Da alle bisher beschriebenen Mutationen die Primärstruktur der SET-Domäne unterbrechen, liegt die Vermutung nahe, daß es die SET-Domäne als solche ist, die eine entscheidende Rolle im Übergang vom normalen zum transformierten Zustand spielt. Eine wichtige Funktion für die SET-Domäne kann ferner aufgrund ihrer evolutionären Konservierung in Genprodukten, die von der Hefe bis zum Menschen auftreten, vermutet werden.

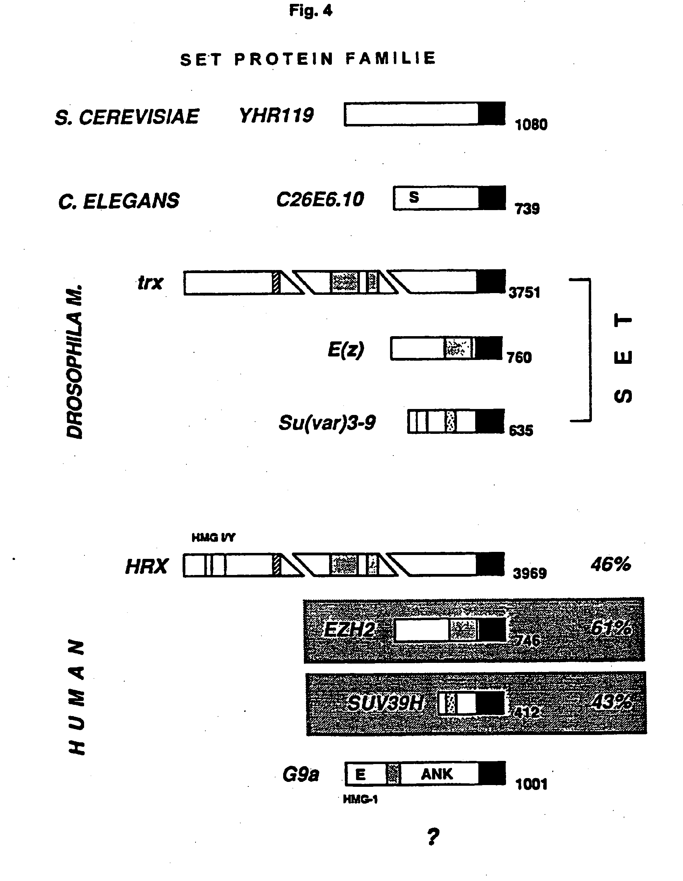

Fig. 4 zeigt die evolutionäre Konservierung von SET-Domäne-Proteinen: Unter Verwendung des tfasta-Programms des Wisconsin GCG Netzwerkservice wurden Proteine und offene Leserahmen mit Homologie zur SET-Domäne identifiziert. In der Figur ist eine repräsentative Auswahl von Hefe bis zum Menschen gezeigt. Ziffern geben Aminosäuren an. Die Carboxyterminale SET-Domäne ist durch eine schwarze Box dargestellt, Cys-reiche Regionen durch eine dunkel gepunktete Box, das GTP-Bindungsmotiv in *Su*(*var*)3-9 durch eine hellgepunktete Box und die Chromo-Domäne von *Su*(*var*)3-9 und *H3-9* durch eine offene Box mit hellen Punkten. Eine Region mit Homologie zu Methyltransferase (*trx* und *HRX*) ist als schraffierte Box dargestellt. A/T-"Haken" ("A/T Hooks") sind durch vertikale Linien dargestellt. Eine weitere Ser-reiche Region (S in C26E6.10) und eine Glu-reiche Region (E in G9a) oder Ankyrin-Repeats (ANK in G9a) sind ebenfalls hervorgehoben. YHR119 (GeneBank Accession No. U00059) und C26E6.10 (GeneBank Accession No. U13875) sind offene Leserahmen von kürzlich in die Datenbank eingetragenen Cosmiden ohne eine funktionelle Charakterisierung. Prozente geben die Gesamtheit der Aminosäure-Identitäten zwischen den humanen und den *Drosophila*-Proteinen an.

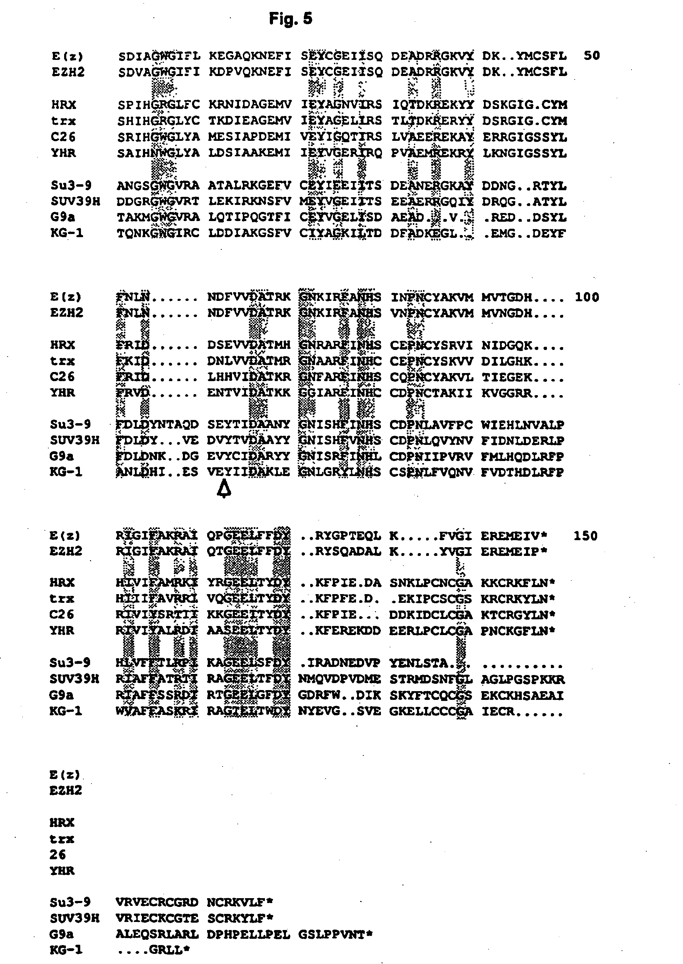

Fig. 5 zeigt die Übereinstimmung an Aminosäuren der SET-Domäne. Die SET-Domäne der in Fig. 4 dargestellten Gene wurde unter Verwendung des Pileup-Programms des Wisconsin GCG Netzwerkservice angeordnet. Für den Vergleich der *KG-1*-SET-Domäne wurde vor dem Pileup die große Aminosäure-Insertion, die die SET-Domäne in zwei Hälften spaltet, entfernt. Aminosäure-Positionen, die 8 von 10 Übereinstimmungen aufweisen, sind hervorgehoben.

Aufgrund der im Rahmen der vorliegenden Erfindung festgestellten Kriterien erweist sich eine Beteiligung der Gene, die eine SET-Domäne haben, an der chromatinabhängigen Entstehung der deregulierten Proliferation; diese Gene bzw. die davon abgeleiteten cDNAs sowie Teil- und mutierte Sequenzen können somit bei der Therapie und Diagnose von Erkrankungen, die auf eine derartige Proliferation zurückzuführen sind, eingesetzt werden:

Unterschiede im Transkriptionsniveau von SET-Domäne-RNAs zwischen normalen und transformierten Zellen können als diagnostischer Parameter für Erkrankungen herangezogen werden, in denen die Expression von SET-Domäne-Genen dereguliert ist:

So können Oligonukleotide, kodierend für die SET-Domäne als solche bzw. Teilabschnitte davon, als diagnostischer Marker verwendet werden, um bestimmte Krebsarten, in denen die SET-Domäne mutiert ist, zu diagnostizieren. Für die detaillierte Analyse der Funktion der erfindungsgemäßen cDNAs oder Abschnitten davon im Hinblick auf den diagnostischen Einsatz von SET-Domäne-Gensequenzen wurden im Rahmen der vorliegenden Erfindung die homologen Maus-cDNAs von *EZH1* (*Ezh1*) und *SUV39H* (*Suv39h*) isoliert. Bei Verwendung einer Maus-spezifischen, für die SET-Domäne kodierenden, DNA-Sonde in "RNAse protection" Analysen zur Untersuchung der *Ezh1*-Genaktivität während der normalen Mausentwicklung zeigte sich ein eher breites Expressionsprofil, das ähnlich dem von *bmi* (Haupt et al., 1991) ist. Die mit den Maussequenzen durchgeführten Analysen werden mit Humansequenzen ausgeweitet, um die RNA-Mengen zwischen unreifen Vorläuferzellen, Tumorzellen und differenzierten Zellen in verschiedenen humanen Zellkultursystemen zu vergleichen. Um festzustellen, ob sich die SET-Domäne dementsprechend als diagnostischer Tumormarker für spezifische Krebserkrankungen oder als genereller diagnostischer Parameter eignet, können gängige Methoden zur Bestimmung der RNA-Konzentration, die in einschlägigen Laborhandbüchern (Sambrook, J., Fritsch, E.F. und Maniatis, T., 1989, Cold Spring Harbor Laboratory Press) beschrieben sind, wie Northern Blot, S1-Nuclease-Protection-Analyse oder RNAse-Protection-Analyse, verwendet werden.

Um die Häufigkeit zu untersuchen, mit der die SET-Domäne spezifischen Mutationen unterliegt, können die SET-spezifischen DNA-Sonden zur Analyse von Einzelstrang-Konformationspolymorphismen (single strand conformation polymorphisms; SSCP; Gibbons et al., 1995) eingesetzt werden.

Krebsarten, bei denen SET-spezifische DNA-Sonden als diagnostischer Marker verwendet werden können, sind Brustkrebs (*EZH1*; Friedman et al., 1994), Synovialksarkom (*SUV39H*; Geraghty et al.; 1993) und Leukämien.

Aufgrund der Kenntnis der Nukleotidsequenz der SET-Domäne-Gene können die entsprechenden von der cDNA-Sequenz abgeleiteten Proteine, die ebenfalls Gegenstand der vorliegenden Erfindung sind, in rekombinanter Form herstellt werden, indem die dafür kodierenden cDNAs in geeignete Vektoren inseriert und in Wirtsorganismen exprimiert werden. Die für die Produktion rekombinanter Proteine verwendeten Techniken sind dem Durchschnittsfachmann geläufig und können einschlägigen Handbüchern (Sambrook, J., Fritsch, E.F. und Maniatis, T., 1989, Cold Spring Harbor Laboratory Press) entnommen werden.

Gegenstand der vorliegenden Erfindung sind somit in einem weiteren Aspekt rekombinante DNA-Moleküle, enthaltend die für *EZH2, SUV39H* oder *EZH1* kodierende DNA sowie damit funktionell verbundene Expressionskontrollsequenzen, sowie die damit transformierten Wirtsorganismen.

Die erfindungsgemäßen rekombinanten Proteine können eingesetzt werden, um die Wechselwirkung von SET-Domäne-Proteinen mit Chromatin bzw. mit anderen Mitgliedern von Heterochromatinkomplexen zu analysieren; ausgehend von den dabei erhaltenen Erkenntnissen über die Wirkungsweise dieser Komplexe werden die sich im einzelnen ergebenden Möglichkeiten für das gezielte Eingreifen in die daran beteiligten Mechanismen definiert und können für therapeutische Anwendungen genutzt werden.

Untersuchungen, die der weiteren Analyse der Funktion der SET-Domäne dienen, werden z.B. durchgeführt, indem cDNAs, kodierend für humanes *EZH2* bzw. *SUV39H* und versehen mit einem Epitop, gegen das Antikörper zur Verfügung stehen, *in vitro* sowie in Gewebekulturen exprimiert werden. Nach Immunpräzipitation mit den jeweiligen epitopspezifischen Antikörpern kann festgestellt werden, ob *EZH2* und *SUV39H* miteinander *in vitro* wechselwirken können und ob *in vivo* eine Komplexbildung zwischen *EZH2* und/oder *SUV39H* mit weiteren Chromatinregulatoren stattfindet.

Es wurde bereits von anderen Autoren angenommen (DeCamillis et al., 1992; Rastelli et al., 1993; Orlando und Paro, 1993), daß eine Komplexbildung zwischen verschiedenen Mitgliedern von Heterochromatinproteinen wesentlich für deren Funktion ist. Aufgrund der Verfügbarkeit der erfindungsgemäßen SET-Domäne-Gene kann festgestellt werden, ob die SET-Region eine Domäne darstellt, die aufgrund von Wechselwirkungen funktioniert, oder ob sie zum Entstehen multimerer heterochromatischer Komplexe beiträgt. Ebenso kann festgestellt werden, ob die SET-Domäne eine inhibitorische Funktion hat, ähnlich der aminoterminalen BTB-Domäne verschiedener Chromatinregulatoren, einschließlich des GAGA-Faktors (Adams et al., 1992). Insgesamt erlauben die Analysen von Wechselwirkungen mit Epitop-versehenen *EZH2-* und *SUV39H*-Proteinen eine weitere Charakterisierung der Funktion der SET-Domäne. Dadurch werden Möglichkeiten eröffnet, gegen die deregulierte Aktivität vorzugehen, indem z.B. mittels gentherapeutischer Methoden dominant-negative Varianten der SET-Domäne-cDNA-Sequenzen in die Zelle eingeführt werden. Derartige Varianten werden z.B. erhalten, indem zunächst die funktionellen Domänen der SET-Proteine definiert werden, z.B. die für die DNA/Chromatin- oder die für die Protein/Protein-Wechselwirkung verantwortlichen Sequenzabschnitte, und indem dann die um die jeweilige(n) Domäne(n) oder um Abschnitte davon verkürzten DNA-Sequenzen in der betroffenen Zelle zur Expression gebracht werden, um eine durch das intakte funktionelle Protein verursachte Deregulation der Proliferation zu kompetitieren.

Die Verfügbarkeit der erfindungsgemäßen cDNAs erlaubt ferner die Herstellung transgener Tiere, z.B. Mäuse, in denen SET-Domäne-Gene entweder überexprimiert werden können ("gain-of-function") oder in denen diese Gene ausgeschaltet werden können ("loss-of-function"); für letztere Analysen werden die korrespondierenden Tiersequenzen, insbesondere Maussequenzen, der erfindungsgemäßen Gene eingesetzt. Diese Mäuse sind ebenfalls Gegenstand der vorliegenden Erfindung.

Insbesondere gibt die "gain-of-function"- Analyse, bei der Allele der erfindungsgemäßen Gene in die Maus eingeführt werden, letztlich Aufschluß über die ursächliche Beteiligung von *EZH2* und *SUV39H* an der chromatinabhängigen Förderung der Tumorentstehung. Für die "gain-of-function"- Analyse können die kompletten cDNA-Sequenzen von humanem *EZH2* und *SUV39H* sowie deren mutierte Versionen, wie *EZH1*/*B52* und *MG-44*, mittels Vektoren, die hohe Expressionsraten ermöglichen, z.B. Plasmiden mit dem humanen β-Actin-Promotor, getrieben sowohl vom Enhancer der schweren Kette von Immunglobulinen (Eµ) als auch von Moloney-Virus-Enhancern (Mo-LTR). Kürzlich wurde gezeigt, daß die Eµ/Mo-LTR-abhängige Überexpression des *bmi*-Gens, welches gemeinsam mit *EZH2* zur *Pc*-Gruppe negativer Chromatinregulatoren zählt, ausreicht in transgenen Mäusen Lymphome zu erzeugen (Alkema et al., 1995).

Indem in "loss-of-function"-Analysen die endogenen Maus-Loci für *Ezh1* und *Suv39h* durch homologe Rekombination in embryonalen Stammzellen unterbrochen werden, kann bestimmt werden, ob der Verlust der *in vivo*-Genfunktion zu einer abnormalen Entwicklung der Maus führt.

Aufgrund dieser *in vivo*-Systeme kann die Wirkung von *EZH2* und *SUV39H* bestätigt werden; diese Systeme dienen außerdem als Grundlage für Tiermodelle im Hinblick auf eine humane Gentherapie.

Der gentherapeutische Einsatz der erfindungsgemäßen DNA-Sequenzen oder davon abgeleiteter Sequenzen (z.B. komplementärer Antisenseoligonukleotide) erfolgt - je nachdem, ob die zu behandelnde Erkrankung auf eine Deregulation von Chromatin infolge des Fehlens der funktionellen Gensequenz, oder aber infolge einer Überexpression der entsprechenden Gene zurückzuführen ist - durch Einführung der funktionellen Gensequenz, durch Inhibierung der Genexpression, z.B. mit Hilfe von Antisenseoligonukleotiden, oder durch Einführung einer für eine dominant-negative Mutante kodierenden Sequenz. Die Einführung der jeweiligen DNA-Sequenzen in die Zelle kann mit Hilfe von Standardmethoden für die Transfektion höherer eukaryotischer Zellen erfolgen, zu denen der Gentransfer mittels viraler Vektoren (Retrovirus, Adenovirus, Adeno-assoziiertes Virus) oder mittels nicht-viralen Systemen auf Basis der Rezeptor-vermittelten Endozytose zählen; Übersichten über gebräuchliche Methoden werden z.B. von Mitani und Caskey, 1993; Jolly, 1994; Vile und Russel, 1994; Tepper und Mule, 1994; Zatloukal et al., 1993, WO 93/07283 gegeben.

Für eine Inhibierung der Expression der erfindungsgemäßen Gene kommen auch niedermolekulare Substanzen in Betracht, die in die Transkriptionsmaschinerie eingreifen; nach Analyse der 5'-regulatorischen Region der Gene kann nach Substanzen gescreent werden, die die Wechselwirkung der jeweiligen Transkriptionsfaktoren mit dieser Region ganz oder teilweise blockieren, z.B. mit Hilfe der in der WO 92/13092 beschriebenen Methode.

Eine Inhibierung der deregulierten Proliferation kann auch am Genprodukt ansetzen, indem die entsprechenden Antikörper gegen das *EZH2-* oder *SUV39H*-Protein, vorzugsweise humane oder humanisierte Antikörper, therapeutisch eingesetzt werden. Die Herstellung solcher Antikörper erfolgt nach bekannten Methoden, wie sie z.B. von Malavsi und Albertini, 1992, oder von Rhein, 1993, beschrieben wurden.

Antikörper gegen *EZH2* oder *SUV39H*, die therapeutisch oder diagnostisch verwendet werden können, sind ebenfalls Gegenstand der vorliegenden Erfindung.

### Figurenübersicht

- Fig. 1:: Aminosäure-Sequenzvergleich zwischen *EZH2* und *E*(*z*)
- Fig. 2:: Aminosäure-Sequenzvergleich zwischen *SUV39H* und *Su*(*var*)3-9
- Fig. 3:: Aberrante Transkripte von humanen SET-Domäne-Genen
- Fig. 4:: Evolutionäre Konservierung von SET-Domäne-Proteinen
- Fig. 5:: Aminosäure-Übereinstimmung in der SET-Domäne
- Fig. 6:: DNA- und Aminosäure-Sequenz von *EZH2*
- Fig. 7:: DNA- und Aminosäure-Sequenz von *SUV39H*
- Fig. 8:: partieller Sequenzvergleich zwischen den cDNAs von humanem *EZH2* und *EZH1*

### Beispiel

### a) Herstellung einer cDNA-Bibliothek

Es wurde eine humane B-Zell-spezifische cDNA-Bibliothek, wie von Bardwell und Treisman, 1994, beschrieben, hergestellt, indem Poly(A)⁺-RNA aus humanen BJA-B-Zellen isoliert, mittels Poly(dT)₁₅-Priming revers transkribiert und in doppelsträngige cDNA konvertiert wurde. Nach Zugabe eines EcoRI-Adapters der Sequenz 5' AATTCTCGAGCTCGTCGACA wurde die cDNA in die EcoRI-Stelle des Bakteriophagen gt10 ligiert. Die Propagierung und Amplifizierung der Bibliothek erfolgte in E.coli C600.

### b) Herstellung von DNA-Sonden

*Drosophila*-DNA-Sonden, kodierend für die konservierten SET-Domänen von *E*(*z*) und *Su*(*var*)3-9, wurden auf der Grundlage der publizierten *Drosophila*-Sequenzen (Jones und Gelbart, 1993; Tschiersch et al., 1994) mittels Polymerasekettenreaktion (PCR) hergestellt: 1 µg von *Drosophila melanogaster*-DNA (Clontech) wurde mit den beiden Primern *E*(*z*) 1910 (5'ACTGAATTCGGCTGGGGCATCTTTCTTAAGG) und *E*(*z*) 2280 (5' ACTCTAGACAATTTCCATTTCACGCTCTATG) einer PCR-Amplifikation (35 Zyklen zu 30 sek bei 94°C, 30 sek bei 55°C und 30 sek bei 72°C) unterworfen. Die entsprechende SET-Domäne-Sonde für *Su*(*var*)3-9 wurde von 10 ng Plasmid-DNA (Tschiersch et al., 1994; Klon M4) mit dem Primerpaar suvar.up (5' ATATAGTACTTCAAGTCCATTCAAAAGAGG) und suvar.dn (5' CCAGGTACCGTTGGTGCTGTTTAAGACCG) amplifiziert, wobei dieselben Zyklusbedingungen verwendet wurden. Die erhaltenen SET-Domäne-DNA-Fragmente wurden Gelgereinigt und teilsequenziert, um die Richtigkeit der amplifizierten Sequenzen zu bestätigen.

### c) Screenen der cDNA-Bibliothek

5 x 10⁵ Plaque-bildende Einheiten ("plaque forming units", pfu) wurden mit 5 ml Kultur des bakteriellen Wirtsstammes E.coli C600 (bei einer optischen Dichte OD₆₀₀ von 0.5 in 10 mM MgSO₄ suspendiert) bei 37°C 15 min lang inkubiert und dann auf eine große (200 mm x 200 mm) vorgewärmte LB-Schale ausgegossen. Nach Wachstum über Nacht bei 37°C wurden die Phagen auf einer Nylonmembran (GeneScreen) absorbiert. Die Membran wurde, die Seite mit den absorbierten Phagen nach oben schauend, 30 sek lang in Denaturierungslösung (1.5 M NaCl, 0.5 M NaOH) schwimmen gelassen, dann 60 sek in Denaturierungslösung eingetaucht und abschließend 5 min lang in 3 M NaCl, 0.5 M Tris pH 8, neutralisiert. Dann wurde die Membran kurz in 3xSSC gespült und die Phagen-DNA mittels UV-Vernetzung auf das Nylonfilter fixiert. Das Filter wurde 30 min lang bei 50°C in 30 ml Church-Puffer (1 % BSA, 1 mM EDTA und 0.5 M NaHPO₄, pH 7.2) prähybridisiert, anschließend wurden 2 x 10⁶ cpm der radioaktiv markierten DNA-Sondenmischung (E(z)-SET und *Su*(*var*)3-9-SET) zugegeben; die DNA-Sonden wurden durch Random-priming unter Verwendung des RediPrime-Kit (Amersham) hergestellt. Die Hybridisierung wurde über Nacht bei 50°C durchgeführt. Nach Entfernung der Hybridisierungslösung wurde das Filter 10 sek in 2xSSC, 1 % SDS bei Raumtemperatur gewaschen, anschließend wurde 10 sek lang bei 50°C gewaschen. Das Filter wurde in Saranwrap gewickelt und unter Verwendung einer Verstärkerfolie der Autoradiographie unterworfen.

Positive Phagenkolonien wurden auf der Originalplatte mittels Zuordnung des Autoradiogramms identifiziert und die entsprechenden Agarstückchen mit dem größeren Ende einer Pasteur-Pipette entfernt. Der Phagen-Pool wurde über Nacht bei 4°C in 1 ml SM-Puffer (5,8 g NaCl, 2 g MgSO₄-H₂O, 50 ml Tris pH 7.5, 5 ml 2 %ige Gelatine auf 1 l H₂O), enthaltend einige Tropfen CHCl₃, eluiert. Das Phagenlysat wurde für eine zweite und dritte Screeningrunde wieder ausplattiert um einzelne, gut isolierte positive Plaques (20 bis 100 Plaques pro Platte in der dritten Runde) zu erhalten.

### d) Sequenzanalyse

Die cDNA-Inserts von rekombinanten Phagen wurden in den Polylinker von pBluescript KS (Stratagene) subkloniert und auf einem automatischen Sequenzer (Applied Biosystems) unter Verwendung der Didesoxy-Methode sequenziert. Die komplette Sequenz von wenigstens zwei unabhängigen Isolaten pro erhaltenen Gen wurde mittels "Primer walking" ermittelt. Die Sequenzen wurden mit dem GCG-Software-Paket (University of Wisconsin) analysiert, die Untersuchung auf Homologie wurde mit dem "Blast and fasta" oder "tfasta" Netzwerkservice durchgeführt. Die kompletten Sequenzen von *EZH2* und *SUV39H* sind in Fig. 6 und 7 dargestellt.

### Literatur

Adams et al., 1992, Genes & Dev. 6, 1589-1607.
Alkema et al., 1995, Nature 374, 724-727.
Allshire et al., 1994, Cell 76, 157-169.
Bardwell und Treisman, 1994, Genes & Dev. 8, 1644-1677.
Brunk et al., 1991, Nature 353, 351-355.
Buck und Shore, 1995, Genes & Dev. 9, 370-384.
Cleary, 1991, Cell 66, 619-622.
Counter et al., 1992, Embo J. 11, 1921-1928.
DeCamillis et al., 1992, Genes & Dev. 6, 223-232.
Eissenberg et al., 1992, Genetics 131, 345-352.
Friedman et al., 1994, Cancer Research 54, 6374-6382.
Garzino et al., 1992, Embo J. 11, 4471-4479.
Geraghty et al., 1993, Genomics 16, 440-446.
Gibbons et al., 1995, Cell 80, 837-845.
Gu et al., 1992, Cell 71, 701-708.
Haupt et al., 1991, Cell 65, 753-763.
Jolly, D., 1994, Cancer Gene Therapy 1, 51.
Jones und Gelbart, 1993, MCB 13 (10), 6357-6366.
Kennedy et al., 1995, Cell 80, 485-496.
Locke et al., 1988, Genetics 120, 181-198.
Malavsi, F. und Albertini, A., 1992, TIBTECH 10, 267-269.
Messmer et al., 1992, Genes & Dev. 6, 1241-1254.
Milner und Campbell, 1993, Biochem. J. 290, 811-818.
Mitäni, K. und Caskey, C.T., 1993, Trends in Biotechnology 11, 162-166.
Nomura et al., 1994, Unpublished. GeneBank accession number: D31891.
Orlando und Paro, 1993, Cell 75, 1187-1198.
Pardue, 1991, Cell 66, 427-431.
Rastelli et al., 1993 Embo J. 12, 1513-1522.
Renauld et al., 1993, Genes & Dev. 7, 1133-1145.
Reuter und Spierer, 1992, BioEssays 14, 605-612.
Rhein, R., 1993, The Journal of NIH Res. 5, 40-46.
Smouse und Perrimon, 1990, Dev. Biol. 139, 169-185.
Solomon et al., 1991, Science 254, 1153-1160.
Tepper, R.I. und Mule, J.J., 1994, Human Gene Therapy 5, 153.
Tkachuk et al., 1992, Cell 71, 691-700.
Tschiersch et al., 1994, Embo J. 13 (16), 3822-3831.
Vile, R. und Russel S., 1994, Gene Therapy 1, 88.
Zatloukal, K., Schmidt, W., Cotten, M., Wagner, E., Stingl, G. und Birnstiel, M.L., 1993, Gene 135, 199.

## Patentansprüche

1. DNA-Molekül, kodierend für ein humanes Chromatinregulator-Protein oder für eine Teilsequenz davon, die der SET-Domäne entspricht, **dadurch gekennzeichnet, dass** es ausgewählt ist aus der Gruppe von DNA-Molekülen, welche die in Fig. 6 bzw. Fig. 7 dargestellte Nukleotidsequenz aufweisen, kodierend für Proteine der Bezeichnung EZH2 bzw. SUV39H, oder Abschnitte dieser Sequenz, die der SET-Domäne von EZH2 bzw. SUV39H entsprechen.

2. DNA-Molekül nach Anspruch 1, **dadurch gekennzeichnet, daß** es ein cDNA-Molekül ist, das für funktionelles humanes EZH2 bzw. SUV39H kodiert, einschließlich seiner degenerierten Varianten sowie einer EZH2-Variante der Bezeichnung EZH1, die als Teilsequenz die Sequenz gemäß Fig. 8 enthält.

3. DNA-Molekül nach Anspruch 1, **dadurch gekennzeichnet, daß** es ein cDNA-Molekül ist, das für EZH2 kodiert.

4. DNA-Molekül nach Anspruch 1, **dadurch gekennzeichnet, daß** es ein cDNA-Molekül ist, das für die SET-Domäne von EZH2 kodiert.

5. DNA Molekül nach Anspruch 1, **dadurch gekennzeichnet, daß** es ein cDNA-Molekül ist, das für SUV39H kodiert.

6. DNA-Molekül nach Anspruch 1, **dadurch gekennzeichnet, daß** es ein cDNA-Molekül ist, das für die SET-Domäne von SUV39H kodiert.

7. Rekombinantes DNA-Molekül, enthaltend ein in Anspruch 3 oder 5 definiertes cDNA-Molekül, funktionell verbunden mit Expressionskontrollsequenzen, zur Expression in prokaryotischen oder eukaryotischen Wirtsorganismen

8. Prokaryotische oder nicht-humane eukaryotische Wirtsorganismen, transformiert mit einem rekombinanten DNA-Molekül nach Anspruch 7.

9. Rekombinantes humanes Chromatinregulator-Protein EZH2 oder dessen SET-Domäne, erhältlich durch Expression eines in Anspruch 3 oder 4 definierten cDNA-Moleküls.

10. Rekombinantes humanes Chromatinregulator-Protein SUV39H oder dessen SET-Domäne, erhältlich durch Expression eines in Anspruch 5 oder 6 definierten cDNA-Moleküls.

11. Antikörper gegen ein Protein der Bezeichnung EZH2, das die in Fig. 6 dargestellte Sequenz aufweist.

12. Antikörper gegen ein Protein der Bezeichnung SUV39H, das die in Fig. 7 dargestellte Sequenz aufweist.

13. cDNA-Molekül nach Anspruch 4 oder 6 zur Behandlung und Diagnose von Erkrankungen des Menschen, die auf eine Deregulation von Chromatinregulator-Genen, die eine SET-Domäne aufweisen, zurückzuführen sind.

14. Antikörper nach Anspruch 11 oder 12 zur Behandlung und Diagnose von Erkrankungen des Menschen, die auf eine Deregulation von Chromatinregulator-Genen, die eine SET-Domäne aufweisen, zurückzuführen sind.

## Claims

1. DNA molecule coding for a human chromatin regulator protein or for a partial sequence thereof that corresponds to the SET domain, **characterised in that** it is selected from among the DNA molecules that include the nucleotide sequence shown in Fig. 6 or 7, coding for proteins designated EZH2 and SUV39H, respectively, or fragments of this sequence that correspond to the SET domain of EZH2 or SUV39H.

2. DNA molecule according to claim 1, **characterised in that** it is a cDNA molecule that codes for functional human EZH2 or SUV39H, including the degenerate variants thereof and an EZH2 variant designated EZH1, which contains as partial sequence the sequence according to Fig. 8.

3. DNA molecule according to claim 1, **characterised in that** it is a cDNA molecule that codes for EZH2.

4. DNA molecule according to claim 1, **characterised in that** it is a cDNA molecule that codes for the SET domain of EZH2.

5. DNA molecule according to claim 1, **characterised in that** it is a cDNA molecule that codes for SUV39H.

6. DNA molecule according to claim 1, **characterised in that** it is a cDNA molecule that codes for the SET domain of SUV39H.

7. Recombinant DNA molecule containing a cDNA molecule as defined in claim 3 or 5, functionally linked to expression control sequences, for expression in prokaryotic or eukaryotic host organisms.

8. Prokaryotic or non-human eukaryotic host organisms, transformed with a recombinant DNA molecule according to claim 7.

9. Recombinant human chromatin regulator protein EZH2 or the SET domain thereof, obtainable by expression of a cDNA molecule as defined in claim 3 or 4.

10. Recombinant human chromatin regulator protein SUV39H or the SET domain thereof, obtainable by expression of a cDNA molecule as defined in claim 5 or 6.

11. Antibody against a protein designated EZH2 which comprises the sequence shown in Fig. 6.

12. Antibody against a protein designated SUV39H which comprises the sequence shown in Fig. 7.

13. cDNA molecule according to claim 4 or 6 for the treatment and diagnosis of diseases in man which can be traced to deregulation of chromatin regulator genes that comprise an SET domain.

14. Antibody according to claim 11 or 12 for the treatment and diagnosis of diseases in man which can be traced to deregulation of chromatin regulator genes that comprise an SET domain.

## Revendications

1. Molécule d'ADN codant une protéine de régulation de la chromatine humaine ou une séquence partielle de celle-ci, qui correspond au domaine SET, **caractérisée en ce qu'**elle est choisie dans le groupe des molécules d'ADN qui comportent la séquence nucléotidique représentée sur la figure 6 ou la figure 7, codant des protéines de dénomination EZH2 ou SLTV39H, ou des segments de cette séquence qui correspondent au domaine SET de EZH2 ou SUV39H.

2. Molécule d'ADN selon la revendication 1 **caractérisée en ce que** c'est une molécule d'ADNc qui code EZH2 ou SUV39H humaine fonctionnelle, y compris ses variants dégénérés ainsi qu'un variant de EZH2 de dénomination EZH1 qui contient comme séquence partielle la séquence selon la figure 8.

3. Molécule d'ADN selon la revendication 1 **caractérisée en ce que** c'est une molécule d'ADNc qui code EZH2.

4. Molécule d'ADN selon la revendication 1 **caractérisée en ce que** c'est une molécule d'ADNc qui code le domaine SET de EZH2.

5. Molécule d'ADN selon la revendication 1 **caractérisée en ce que** c'est une molécule d'ADNc qui code SUV39H.

6. Molécule d'ADN selon la revendication 1 **caractérisée en ce que** c'est une molécule d'ADNc qui code le domaine SET de SUV39H.

7. Molécule d'ADN recombinant contenant une molécule d'ADNc définie dans la revendication 3 ou 5, liée fonctionnellement à des séquences de contrôle de l'expression, pour l'expression dans des organismes hôtes procaryotes ou eucaryotes.

8. Organismes hôtes procaryotes ou eucaryotes non humains transformés avec une molécule d'ADN recombinant selon la revendication 7.

9. Protéine de régulation de la chromatine humaine recombinante EZH2 ou son domaine SET pouvant être obtenu(e) par expression d'une molécule d'ADNc définie dans la revendication 3 ou 4.

10. Protéine de régulation de la chromatine humaine recombinante SUV39H ou son domaine SET pouvant être obtenu(e) par expression d'une molécule d'ADNc définie dans la revendication 5 ou 6.

11. Anticorps contre une protéine de dénomination EZH2 qui comporte la séquence représentée sur la figure 6.

12. Anticorps contre une protéine de dénomination SUV39H qui comporte la séquence représentée sur la figure 7.

13. Molécule d'ADNc selon la revendication 4 ou 6 pour le traitement et le diagnostic des maladies de l'homme qui sont à attribuer à une dérégulation des gènes de régulation de la chromatine qui comportent un domaine SET.

14. Anticorps selon la revendication 11 ou 12 pour le traitement et le diagnostic des maladies de l'homme qui sont à attribuer à une dérégulation des gènes de régulation de la chromatine qui comportent un domaine SET.
